# EUROPEAN PATENT SPECIFICATION

(11) **EP 3 901 626 B1**
(45) Date of publication and mention of the grant of the patent: **04.06.2025**
(21) Application number: 19856447.8
(22) Date of filing: 18.12.2019
(51) Int. Cl.: G01N 27/49

(54) **AMPEROMETRIC SENSOR WITH FOUR ELECTRODES**
AMPEROMETRISCHER SENSOR MIT VIER ELEKTRODEN
CAPTEUR AMPEROMETRIQUE AVEC QUATRE ELECTRODES

(30) Priority: 18.12.2018 ES 201831238
(43) Date of publication of application: 27.10.2021
(73) Proprietor: MICRODOS S.R.L., 02100 Vazia (RI) (IT)
(72) Inventor: CORBELLA CORDOMÍ, Xavier Maria, 08130 Santa Perpetua de la Mogoda (ES); HERNÁNDEZ ARTESEROS, José Alberto, 08130 Santa Perpetua de la Mogoda (ES); MARTÍNEZ GARCÍA, Tomás Antonio, 08130 Santa Perpetua de la Mogoda (ES)
(74) Representative: Marks & Clerk LLP
(86) International application number: PCT/ES2019/070859
(87) International publication number: WO 2020/128129

(56) References cited:
- WO-A1-2017/020133
- US-A- 3 862 895
- US-A1- 2008 283 399
- US-A1- 2009 014 329
- US-A1- 2014 083 865
- US-B2- 6 894 502
- US-B2- 8 142 641
- DOSING PUMPS ITC: "Cl2 SENSOR 420MB", 3 July 2019 (2019-07-03), XP055689723, Retrieved from the Internet <URL:https://www.itc.es/wp-content/uploads/ITC_CL_Sensor_420MB-EN.pdf> [retrieved on 20200428]

## Description

### Field of the invention

The present invention relates to an amperometric sensor for measuring analytes in fluids.

In particular, this analyte is an oxidising agent, more specifically, free chlorine. Also, this fluid is specifically water.

### Background of the invention

Amperometric sensors measure the intensity of current resulting from the oxidation or reduction of an electroactive substance on the surface of an electrode to which a constant potential has been applied.

The appropriate choice of this potential provides a certain electrochemical selectivity, since it produces a reaction of one or another chemical species. This selectivity is often insufficient, and so the surface of the electrodes must be modified (chemically or biologically) to increase the selectivity and sensitivity of the detection.

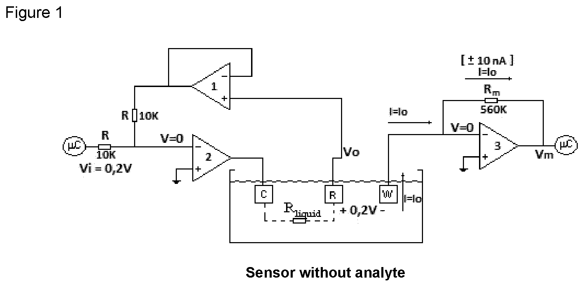

Three-electrode systems are used for amperometric signal measurements (see Figure 1).

The working electrode is the one on which the passage of current is measured once a potential difference has been established between the working electrode and the reference electrode, with the third auxiliary electrode acting as a counter electrode to close the electrical circuit. A potentiostat is also required which, by means of the auxiliary electrode, keeps the potential between the reference electrode and the working electrode constant. The reference electrode is usually made of Ag/AgCl, while the working and auxiliary electrodes are made of conductive and inert materials such as noble metals, carbon derivatives and conductive polymers (see http://webs2002.uab.es/ipividori/TP/TP%2008.pdf)

Currently, these amperometric sensors are used to measure analytes, in particular oxidising agents such as free chlorine. The following electrochemical reaction would take place: 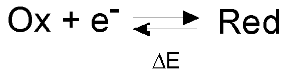 which in the case of free chlorine would correspond to:

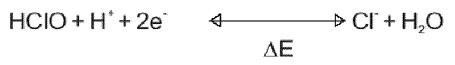

According to this reaction, the current intensity is proportional to the amount of hypochlorous acid in the water.

By setting the working electrode to the specific reduction potential of the analyte the current (IT) from the counter or auxiliary electrode to the working electrode will be modified. The variation of the electric current intensity is a direct measure of the concentration of the analyte.

Document WO-2017/020133 discloses an amperometric sensor for measuring an analyte in a fluid, the sensor comprising: a reference electrode, an auxiliary electrode and a working electrode, and circuitry for maintaining between the reference electrode and the working electrode a potential difference to carry out the electrochemical reaction of the analyte. The working electrode is connected to the inverting input of an operational amplifier, with the non-inverting input of the amplifier being directly connected to ground.

There are currently several problems associated with this type of amperometric sensor.

First of all, there is a high sensitivity to electrical interference.

The generated electrical current is extremely low. This is not a major problem in controlled environments such as a laboratory, but it is a problem in less controlled environments such as water distribution networks, industry, swimming pools, etc. In this other type of environment, electrical noise can be produced in the form of currents or potential differences. The amount of these currents is very significant with respect to the amount of the measurement current, and as the measurement circuit is closed by the water, the value of the amount as read is easily altered by the presence of these currents, giving incorrect readings.

Any presence of electrical noise in the fluid can lead to currents external to the measurement system from the working electrode W to the ground of the amperometric sensor increasing the total current IT = lo (without analytes) + I_{R} (with analytes) + Is (due to noise), and altering the value of the measurement corresponding to the analyte being measured.

As a protection system, in some cases membranes are used to prevent the electrodes from being in direct contact with the water containing the analyte, creating an intermediate chamber with the presence of an electrolyte that allows the circuit to be closed. The selective membrane allows the ions of the oxidising agent, e.g. free chlorine, to pass through and the amperometric reaction takes place in this controlled environment. These membranes slow down the measurement, are very fragile and get dirty easily. The electrolyte deteriorates over time and must be replaced, involving thereby maintenance. Broken or clogged membranes or damaged electrolytes will result in incorrect readings. This makes this type of sensor delicate to use, with necessary maintenance tasks and impossible to use either under pressure (or vacuum) or with solids in suspension.

There is also the problem of the materials and manufacture of the electrodes.

The materials used for amperometric sensors must be such that oxidation is impossible since this would generate an exchange of electrons that would be picked up by the sensor, erroneously increasing the reading of the read current. The materials normally used are gold and platinum. For external fluid environments that do not have an exhaustive control as in a laboratory, it is convenient for the sensors to be robust as well as reliable and, therefore, it is convenient for their electrodes to have a geometry with the largest possible surface in contact with the fluid to be measured and thus obtain a greater intensity of the reading. As extremely expensive materials are used, this is a major drawback.

Finally, there is also the problem of the surface of the electrodes.

Due to the electrochemical reaction of the measurement process, it is common to see a slight oxidation of the gold layer that acts as an electrical insulator, decreasing the intensity of the reading. The risk of damaging the gold surface when applying mechanical cleaning means makes it difficult to remove this layer, which in any case implies manipulating the sensor to remove it from the pipe where it is installed.

In view of the above problems, the present inventors have developed an amperometric sensor which overcomes such disadvantages, as well as a process for manufacturing an optimum working electrode to be used in the amperometric sensor of the present invention.

### Brief description of the drawings

Figure 1 shows a 3-electrode system of the prior art without analyte.
Figure 2 shows a 3-electrode system of the prior art with analyte.
Figure 3 shows a 3-electrode system of the prior art with analyte and external interferences.
Figure 4 shows the 4-electrode system of the present invention with analyte and external interferences.
Figure 5A shows the gold surface of the electrode contaminated with the copper of the core and Figure 5B shows the gold surface of the electrode in perfect conditions.

### Summary of the invention

The present invention relates to an amperometric sensor configured for measuring an analyte in a fluid, characterised in that it has a fourth shunt electrode with a low impedance circuit configured for attracting parasitic currents.

According to examples which are not part of the claimed invention, the present description discloses a process of manufacturing a gold working electrode on a brass core, preferably with a percentage of Cu between 57 and 63 % by weight and of Zn between 36 and 40 % by weight, applicable in the amperometric sensor according to the invention.

### Detailed description of the invention

The present invention relates to an amperometric sensor configured for measuring an analyte in a fluid, as defined in claim 1.

In a preferred embodiment, the sensor further comprises a microcontroller configured for providing a reference electrode with a setpoint voltage.

In another preferred embodiment, the sensor further comprises an ammeter connected between the working electrode and the shunt electrode. Preferably, this ammeter comprises an operational amplifier.

In another preferred embodiment, the sensor further comprises a potentiostat comprising a feedback element to maintain a constant potential, electrically connected to the auxiliary and reference electrodes, and to the microcontroller. Preferably, such a potentiostat feedback element comprises at least one operational amplifier.

In another preferred embodiment, the working electrode comprises a reaction surface configured according to the analyte to be measured, more preferably, this analyte will be an oxidising agent. Preferably, said oxidising agent will be chlorine dioxide, peracetic acid, hydrogen peroxide or free chlorine. More preferably, said oxidising agent is free chlorine, with "free chlorine" being understood to mean any of the chemical forms of molecular chlorine, hypochlorous acid and hypochlorite anion.

In another preferred embodiment, the working electrode is made of platinum or solid gold. By adding a fourth electrode, currents due to electrical noise are diverted and thus prevented from affecting the measurement signal. As the shunt electrode circuit has a much lower impedance than the working electrode (W) (virtual ground), it will conduct non-desired currents (Is) present in the liquid to ground without passing through W, and therefore, will not be measured by the current sensor (A). Thus, the measurement of the analyte is not affected by the presence of these noises, which are common in any water installation.

An example not covered by the present invention relates to a process of manufacturing a gold working electrode on a brass core applicable in the amperometric sensor, according to any of its embodiments, comprising the steps of:
- obtaining a compact nickel (Ni) layer on the brass core, preferably with a Cu percentage between 57 and 63 % by weight and Zn between 36 and 40 % by weight, by means of an electrolytic process;
- applying a gold (Au) base layer for adhesion of the final layer;
- applying a final, homogeneous gold (Au) layer with zero porosity.

In a preferred embodiment, the final gold layer has a depth of up to 10 µm.

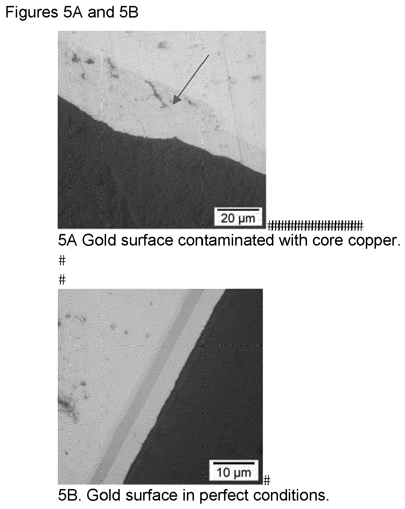

This process allows a large surface area to be achieved at a low cost, as an alternative to a solid gold or platinum electrode. Preferably, the required electrode dimensions to provide an amperometric sensor with sufficient sensitivity for the described working medium will be between 2 and 5 mm in diameter. On the other hand, this type of coating is usually manufactured by electroplating. In the specific case of the amperometric sensor where electrochemical reactions with the surface material take place, it is essential for a good functioning to guarantee a compact layer without pores, without contamination by the core material and maintaining the adherence between the core and the surface. The existence of currents (can be as high as 3000 nA and above) passing through the gold or platinum surface towards the metal core generates migrations of the core material towards the surface, contaminating it, and can cause the outer layer to become detached (see Figure 5A).

At the same time, another advantage is that an automatic electrochemical cleaning of the surface of the working electrodes (W) takes place, independently of the type of the electrode: a potential is periodically generated which causes the reduction of the oxide layer, allowing the surface of the working electrode (Figure 5B) to be recovered. The frequency of these cleanings is configurable by software.

Another example not covered by the present invention relates to a process for measuring an analyte in a fluid, comprising the steps of:
- using the amperometric sensor, according to any of its embodiments;
- applying a software algorithm to compensate the pH.

Preferably, the amperometric sensor as used employs a working electrode obtained according to the manufacturing process described above.

A number of examples will be provided below that are intended to illustrate the invention and in no way limit the scope of the invention, which is established by the attached claims.

### EXAMPLES

### Particular case of chlorine measurement

In the particular case of chlorine measurement, a voltage of -0.2V between the working electrode (W) and the reference electrode (R) is set. Figure 1 shows a detailed outline with the specific values for the case of chlorine without an analyte.

### Operation of the electronics:

### Operational amplifier (AO) 1. Feedback.

The reference electrode is connected to the non-inverting (+) input of the operational 1. In this way, no current enters through this electrode. The inverter input (-) is connected directly to the output of the operational 1, a typical configuration of a voltage follower operational amplifier. As a result, at the output of the operational amplifier 1 we have the same voltage as the reference electrode.

### Operational amplifier (AO) 2. Potentiostat.

This operational (2) works in the inverter configuration. It has its non-inverting input (+) to the shunt electrode (GND), and the inverting input (-) connected to the microcontroller setpoint voltage. The reverse feedback is done through the fluid and the AO 1. In this way, we have the voltage Vo regulated by the voltage Vi, set point of the microcontroller.

### Operational amplifier (AO) 3. Current measurement.

The AO3 functions as a current to voltage converter via the inverting input (-). The working electrode (W), is maintained at virtual 0V (virtual ground) through the AO3, by having the non-inverting (+) input directly to ground. The measured current is a function of the offset current (lo, without analyte) + the analyte current (Ir, difference when adding analyte). This current is converted into the voltage Vm, which is read by the microcontroller.

### (see Figure 1 without analyte)

### Without analyte

The intensity that we read (I) depends only on the intensity that circulates without the presence of analyte (I = lo), and is called Offset Intensity (lo). This is known by each sensor and is used to calculate the current derived from the analyte reaction.

### With analyte

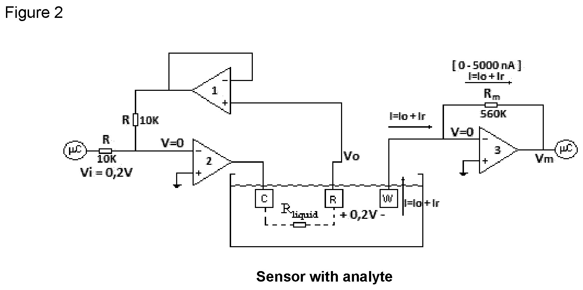

Figure 2 shows how the intensity we read (I = lo + Ir) depends on the intensity that circulates without analyte (lo) and on the intensity produced by the reaction of chlorine (Ir) on the working electrode (W), which is the one we are interested in reading. The current values produced by the chlorine reaction depend on the chlorine concentration and the sensitivity of the sensor, with ranges from 0 to 5000 nA.

### (see Figure 2 with analyte)

### With interference

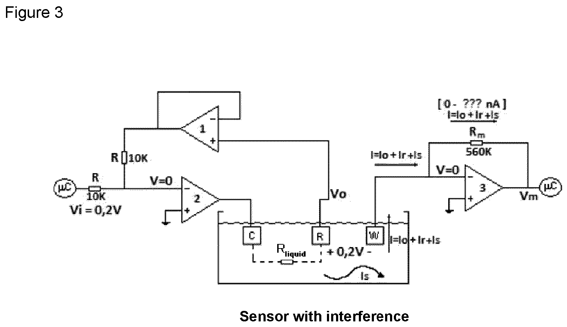

Parasitic currents that can introduce interference to the sensor through the water are usually due to the connection of pumps with variable frequency drives related to the water installation, but can also be due to connections of other equipment that come into contact with the metal pipes. Figure 3 shows how parasitic currents can affect the current reading at AO 3, being added to the current produced by the reaction of the analyte.

### (see Figure 3 with interference)

### With the fourth shunt electrode

As the shunt electrode (G, ground) has a much lower impedance than the working electrode (W, virtual ground), it will conduct the non-desired currents (Is) present in the liquid to ground without passing through the W, and therefore, they will not be measured by the current sensor (AO3). The measurement of the analyte is thus not affected by the presence of these electrical noises.

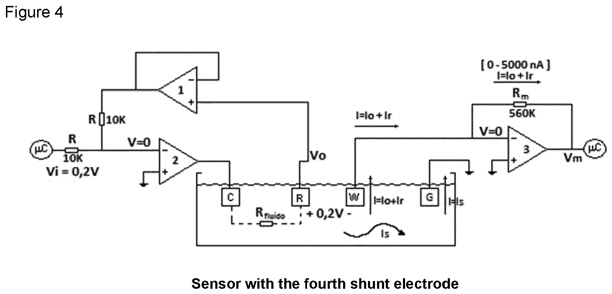

(see Figure 4 with the fourth shunt electrode)

## Claims

1. Amperometric sensor configured for measuring an analyte in a fluid, comprising:
- a first reference electrode (R), a second auxiliary electrode (C) and a third working electrode (W), configured and arranged to be immersed in said fluid when in use,
- a voltage source operationally connected to those first reference electrode (R), second auxiliary electrode (C) and third working electrode (W) to generate and maintain between that first reference electrode (R) and third working electrode (W) a potential difference to carry out the electrochemical reaction of the analyte,
wherein the third working electrode (W) is connected to the inverting input (-) of an operational amplifier (3), having this operational amplifier (3) the non-inverting input (+) directly connected to ground;
**characterised in that** it comprises a fourth shunt electrode (G) having a lower impedance than the third working electrode (W) and connected directly to ground and configured and arranged to be immersed in said fluid when in use.

2. Sensor, according to Claim 1, wherein the sensor comprises a microcontroller configured for providing a set-point voltage to the first reference electrode (R).

3. Sensor, according to any of the preceding claims, wherein the sensor comprises an ammeter connected between the third working electrode (W) and the fourth shunt electrode (G).

4. Sensor, according to any of claims 2 or 3 when depends on 2, wherein the sensor includes a potentiostat comprising a feedback element to maintain a constant potential, electrically connected to the second auxiliary electrode (C) and first reference electrode (R), and to the microcontroller, wherein the feedback element comprises:
- an operational amplifier (2) having the non-inverting input (+) connected to the first reference electrode (R), the output connected to the microcontroller and the inverting input (-) connected to its output, and
- an operational amplifier (1) having the non-inverting input (+) connected to ground, the output connected to the second auxiliary electrode (C) and the inverting input (-) connected to the microcontroller.

5. Sensor, according to any of the preceding claims, wherein the third working electrode (W) comprises a reaction surface configured depending on the analyte to be measured.

6. Sensor, according to Claim 5, wherein the said analyte is free chlorine.

7. Sensor, according to Claim 6, wherein the third working electrode (W) is made of platinum or solid gold.

## Patentansprüche

1. Amperometrischer Sensor, der zum Messen eines Analyten in einem Fluid konfiguriert ist, umfassend:
- eine erste Referenzelektrode (R), eine zweite Hilfselektrode (C) und eine dritte Arbeitselektrode (W), die konfiguriert und angeordnet sind, um im Gebrauch in das Fluid eingetaucht zu werden,
- eine Spannungsquelle, die betriebsmäßig mit der ersten Referenzelektrode (R), der zweiten Hilfselektrode (C) und der dritten Arbeitselektrode (W) verbunden ist, um zwischen der ersten Referenzelektrode (R) und der dritten Arbeitselektrode (W) eine Potentialdifferenz zu erzeugen und aufrechtzuerhalten, um die elektrochemische Reaktion des Analyten auszuführen,
wobei die dritte Arbeitselektrode (W) mit dem invertierenden Eingang (-) eines Operationsverstärkers (3) verbunden ist, wobei bei diesem Operationsverstärker (3) der nicht-invertierende Eingang (+) direkt mit der Erde verbunden ist;
**dadurch gekennzeichnet, dass** er eine vierte Shunt-Elektrode (G) mit einer niedrigeren Impedanz als die dritte Arbeitselektrode (W) umfasst, die direkt mit der Erde verbunden ist und konfiguriert und angeordnet ist, um im Gebrauch in die Flüssigkeit eingetaucht zu werden.

2. Sensor nach Anspruch 1, wobei der Sensor einen Mikrocontroller umfasst, der konfiguriert ist, um eine Sollwertspannung an die erste Referenzelektrode (R) bereitzustellen.

3. Sensor nach einem der vorhergehenden Ansprüche, wobei der Sensor ein Amperemeter umfasst, das zwischen der dritten Arbeitselektrode (W) und der vierten Shunt-Elektrode (G) angeschlossen ist.

4. Sensor nach einem der Ansprüche 2 oder 3, wenn abhängig von Anspruch 2, wobei der Sensor einen Potentiostaten einschließt, der ein Rückkopplungselement zum Aufrechterhalten eines konstanten Potentials umfasst, das elektrisch mit der zweiten Hilfselektrode (C) und der ersten Referenzelektrode (R) und mit dem Mikrocontroller verbunden ist, wobei das Rückkopplungselement umfasst:
- einen Operationsverstärker (2), dessen nicht-invertierender Eingang (+) mit der ersten Referenzelektrode (R) verbunden ist, dessen Ausgang mit dem Mikrocontroller verbunden ist und dessen invertierender Eingang (-) mit dessen Ausgang verbunden ist, und
- einen Operationsverstärker (1), dessen nicht-invertierender Eingang (+) mit der Erde verbunden ist, dessen Ausgang mit der zweiten Hilfselektrode (C) verbunden ist und dessen invertierender Eingang (-) mit dem Mikrocontroller verbunden ist.

5. Sensor nach einem der vorhergehenden Ansprüche, wobei die dritte Arbeitselektrode (W) eine Reaktionsoberfläche umfasst, die in Abhängigkeit von dem zu messenden Analyten konfiguriert ist.

6. Sensor nach Anspruch 5, wobei es sich bei dem Analyten um freies Chlor handelt.

7. Sensor nach Anspruch 6, wobei die dritte Arbeitselektrode (W) aus Platin oder massivem Gold besteht.

## Revendications

1. **Capteur** ampérométrique configuré pour mesurer un analyte dans un fluide, comprenant :
- une première électrode de référence (R), une deuxième électrode auxiliaire (C) et une troisième électrode de travail (W), configurées et agencées pour être immergées dans ledit fluide lors de l'utilisation,
- une source de tension connectée fonctionnellement auxdites première électrode de référence (R), deuxième électrode auxiliaire (C) et troisième électrode de travail (W) pour générer et maintenir entre la première électrode de référence (R) et la troisième électrode de travail (W) une différence de potentiel pour réaliserla réaction électrochimique de l'analyte,
dans lequel la troisième électrode de travail (W) est connectée à l'entrée inverseuse (-) d'un amplificateur opérationnel (3), cet amplificateur opérationnel (3) ayant l'entrée non inverseuse (+) connectée directement à la masse,
**caractérisé en ce qu'**il comprend une quatrième électrode de dérivation (G) présentant une impédance inférieure à la troisième électrode de travail (W) et connectée directement à la masse et configurée et agencée pour être immergée dans ledit fluide lors de l'utilisation.

2. Capteur selon la revendication 1, dans lequel le capteur comprend un microcontrôleur configuré pour fournir une tension de consigne à la première électrode de référence (R).

3. Capteur selon l'une quelconque des revendications précédentes, dans lequel le capteur comprend un ampèremètre connecté entre la troisième électrode de travail (W) et la quatrième électrode de dérivation (G).

4. Capteur selon l'une quelconque des revendications 2 ou 3 lorsqu'elle dépend de la revendication 2, dans lequel le capteur inclut un potentiostat comprenant un élément de rétroaction pour maintenir un potentiel constant, connecté électriquement à la deuxième électrode auxiliaire (C) et à la première électrode de référence (R) et au microcontrôleur, dans lequel l'élément de rétroaction comprend :
- un amplificateur opérationnel (2) dont l'entrée non inverseuse (+) est connectée à la première électrode de référence (R), la sortie est connectée au microcontrôleur et l'entrée inverseuse (-) est connectée à sa sortie, et
- un amplificateur opérationnel (1) dont l'entrée non inverseuse (+) est connectée à la masse, la sortie est connectée à la deuxième électrode auxiliaire (C), et l'entrée inverseuse (-) est connectée au microcontrôleur.

5. Capteur selon l'une quelconque des revendications précédentes, dans lequel la troisième électrode de travail (W) comprend une surface de réaction configurée en fonction de l'analyte à mesurer.

6. Capteur selon la revendication 5, dans lequel ledit analyte est du chlore libre.

7. Capteur selon la revendication 6, dans lequel la troisième électrode de travail (W) est constituée de platine ou d'or massif.
